# EUROPEAN PATENT SPECIFICATION

(11) **EP 2 159 290 B1**
(45) Date of publication and mention of the grant of the patent: **07.03.2012**
(21) Application number: 09168768.1
(22) Date of filing: 27.08.2009
(51) Int. Cl.: C12Q 1/68

(54) **Method of producing microarray having immobilized double-stranded nucleic acid probe including double-stranded region and single-stranded region**
Verfahren zur Herstellung von Mikroarrays mit immobilisierter doppelsträngiger DNA-Sonde einschließlich doppelsträngigem Bereich und einsträngigem Bereich
Procédé de production de micro-réseau doté d'une sonde d'acide nucléique à double brin immobilisé incluant une région à double brin et une région à simple brin

(30) Priority: 27.08.2008 KR 20080084041
(43) Date of publication of application: 03.03.2010
(73) Proprietor: SAMSUNG ELECTRONICS CO., LTD., Suwon-si, Gyeonggi-do (KR)
(72) Inventor: Rhee, Joo-won, Gyeonggi-do (KR)
(74) Representative: Grünecker, Kinkeldey, Stockmair & Schwanhäusser

(56) References cited:
- EP-A1- 1 437 416
- EP-A2- 1 201 769
- WO-A2-00/37683
- WO-A2-99/60156
- WO-A2-2008/069884
- JP-A- 2004 061 170
- US-A1- 2005 136 414
- GE XIJIN ET AL: "Genome-wide analysis of antisense transcription with Affymetrix exon array" BMC GENOMICS, BIOMED CENTRAL, LONDON, GB, vol. 9, no. 1, 22 January 2008 (2008-01-22), page 27, XP021033006 ISSN: 1471-2164
- BARONE A D ET AL: "Photolithographic synthesis of high-density oligonucleotide probe arrays" NUCLEOSIDES, NUCLEOTIDES AND NUCLEIC ACIDS, TAYLOR & FRANCIS, PHILADELPHIA, PA, vol. 20, no. 4/7, 1 April 2001 (2001-04-01), pages 525-531, XP009112168 ISSN: 1525-7770
- PEASE A C ET AL: "LIGHT-GENERATED OLIGONUCLEOTIDE ARRAYS FOR RAPID DNA SEQUENCE ANALYSIS", PROCEEDINGS OF THE NATIONAL ACADEMY OF SCIENCES OF THE UNITED STATES (PNAS), NATIONAL ACADEMY OF SCIENCE, US, vol. 91, 1 May 1994 (1994-05-01), pages 5022-5026, XP000196311, ISSN: 0027-8424, DOI: DOI:10.1073/PNAS.91.11.5022

## Description

### BACKGROUND

### 1. Technical Field

Embodiments of the invention are directed to a method of producing a microarray having an immobilized double-stranded nucleic acid probe including a double-stranded region and a single-stranded region.

### 2. Description of the Related Art

Methods of producing a nucleic acid array by using a photolithography method, also known as a light-directed synthesis process, are well known. These methods involve activating a predefined region of a substrate and then contacting the substrate with a preselected monomer solution. The predefined region may be activated by a light source, typically through a mask. The remainder of the substrate is blocked from the light source by the mask and chemically protected, thus being inert. Thus, it may be determined which region of the substrate is reacted with a monomer according to which region of the substrate is irradiated with a pattern of light. The activation of a predefined region and the contacting of the substrate with another monomer solution are repeatedly performed to form a variety of nucleic acid arrays on the substrate. Other processes, including washing an unreacted monomer solution from the substrate, may be performed, if necessary.

An example of the methods of producing a nucleic acid array by using a light-directed synthesis method will now be described in greater detail. A surface of a solid support, which is selectively modified with a spacer having a photolabile protecting group, such as 6-nitroveratryloxycarbonyl (NVOC) or methyl-6-nitropiperonyloxycarbonyl (MeNPOC), is illuminated through a photolithographic mask, thereby exposing a reactive group (generally a hydroxyl group) in the illuminated region. A 3'-O-phosphoramidite-activated deoxynucleoside protected at the 5'-hydroxyl with a photolabile group is then presented to the surface of the solid support and chemical coupling occurs at sites exposed to light. Following capping and oxidation, the solid support is rinsed and the surface of the solid support is illuminated through a second mask, to expose additional hydroxyl groups for coupling. A second 5'-protected, 3'-O-phosphoramidite-activated deoxynucleoside is presented to the surface of the solid support. The selective photodeprotection and coupling cycles are repeated until the desired set of nucleic acids is obtained. Another example of the light-directed synthesis method discloses that photolabile protecting groups and photolithography are used to achieve light-directed spatially-addressable parallel chemical synthesis of an array of nucleic acids. In this method, computer tools may be used to help the formation of the array.

In such a light-directed synthesis of an array of nucleic acids, a plurality of nucleic acid probes may be synthesized in parallel in spatially-addressable predefined regions. Since the predefined regions are defined by light, the array of nucleic acids may be synthesized by narrowing an interval between the predefined regions, that is, increasing the density of predetermined regions. However, this method includes preparing a new mask for every cycle of a substrate, and thus there is a limitation on the length of nucleic acids that may be synthesized in terms of synthesis efficiency. Synthesis of nucleic acids with a length of 25nt or less is commercially known.

In addition, a method of producing an array of nucleic acids by using a spotting method is known. In the spotting method, a reactant, such as previously synthesized nucleic acids or a nucleic acid monomer, is transferred to a selected region on a substrate such that a relatively small amount of the reactant is directly deposited on the selected region. The deposition may be performed by moving between the selected regions using a dispenser. Examples of dispensers include micropipettes for transferring a monomer solution to a substrate and robot systems for controlling a position of the micropipette with respect to a substrate, or inkjet printers. In addition, the dispenser may be a series of tubes, manifolds, and an array of pipettes, which allow a variety of reagents to be simultaneously transferred to reaction regions. In this method, foreign synthesized nucleic acids are immobilized on a substrate, and thus relatively long nucleic acids may be immobilized on the substrate. However, the immobilization is performed by direct deposition, and the spot density is low.

Xijin Ge et al.: "Genome-wide analysis of antisense transcription with Affymetrix exon array", BMC Genomics, Biomed Central, London, GB, vol. 9, no. 1, 22 January 2008, page 27 describes a method for a genome-wide analysis of antisense transcription with an Affymetrix exon array using a modified cDNA synthesis protocol labeling the targets in reverse orientation as what would be labeled according to the standard protocol.

A. D. Barone, "Photolithographic synthesis of high-density oligonucleotide probe arrays", Nucleotides, Nucleotides 8 Nucleic Acids, Taylor & Francis, Philadelphia, PA, vol. 20, no. 4/7, 1 April 2001, pages 525-531 describes a photolithographic synthesis of high-density oligonucleotide probe arrays. The manufacturing process integrates solid-phase photochemical oligonucleotide synthesis with lithographic techniques. This allows for a light-directed combinatorial oligonucleotide synthesis enabling the large-scale production of oligonucleotide probe arrays consisting of several hundred of thousands oligonucleotide sequences on glass chips.

WO 99/60156 is a Japanese patent application. According to the English abstract as well as an English machine translation of this application, this document appears to disclose a method of using a gene chip, such as a conventional DNA chip having oligonucleotides fixed on a glass slide, proceeding a hybridization reaction with the target gene using the gene chip, and contacting the gene chip with the detection reagent comprising a fluorochromic substance.

JP 2004 061170 A discloses a method and a device for the photolithographic production of oligonucleotides on two-dimensional matrices for the production of so-called DNA, PNA or protein chips, characterized in that a dynamically controlled liquid crystal mask is used as a photolithographic mask.

EP 1 437 416 A1 provides a method for detecting DNA hybridization on a DNA chip, e.g. an Affymetrix gene chip containing a transparent glass chamber, in which a temperature sensor and a thermo-cycler are installed, wherein the temperature is introduced as a fourth detection parameter to determine the melting temperatures by scanning the changes of fluorescence intensity of the fluorescent labels on the double-stranded nucleic acids resulting from the increasing of hybridization temperature.

EP 1 201 769 A2 relates to gene expression monitoring using universal arrays. EP 1 201 769 A2 in particular refers to a method for detecting a plurality of nucleic acids in a target sample comprising to hybridize the sample with a plurality of mediator probes and a plurality of cipher probes immobilized on a substrate. The immobilized cipher probes form the universal array. The mediator probes are used to hybridize with the cipher probes and the target nucleic acid, resulting in the formation of a ternary complex between the target, the mediator probes, and the cipher probes attached to the solid surface.

WO 2008/069884 A2 discloses a two-stage nucleic acid amplification method and systems for performing this method. In the first stage, the nucleic acid of interest is captured on a solid support, for example, through hybridization of a first capture probe on the solid support to a first capture extender and hybridization of the capture extender to a complementary sequence on the nucleic acids of interest. This approach of using a capture probe and a capture extender corresponds to the approach as disclosed in document EP 1 201 769 A2. In the method of WO 2008/069884 A2, subsequently, an amplification multimer, is captured on the solid support by hybridization to the captured target nucleic acid, which amplification multimer can capture a large number of amplification oligomers. After releasing the amplification oligomers accumulated during the first stage, the amplification oligomers act as a target nucleic acid in the second step to accumulate a large number of label probes.

US 2005/0136414 A relates to methods for making locus-specific arrays from universal arrays in situ. The method comprises the steps of (a) providing a universal array having a plurality of assay locations wherein each of the assay locations comprises an adapter probe: (b) providing a plurality of chimeric oligonucleotides: (c) contacting the chimeric oligonucleotides with the adapter probes under conditions for forming a plurality of chimeric-oligonucleotide:adapter hybrids: and (d) converting the hybrids into locus-specific assay locations of the locus-specific array. In one embodiment the universal array is converted into locus specific arrays in situ by direct hybridization to convert the adapter probes into locus-specific probes in situ. In another embodiment the hybrid is extended using a polymerase, which binds at the double-stranded region.

WO 00/37683 relates to to compositions, apparatus and methods useful for concurrently performing multiple high throughput, biological or chemical assays, using repeated arrays of probes. A combination of the invention comprises a surface which comprises a plurality of test regions at least two of which, and in a preferred embodiment, at least twenty of which, are substantially identical wherein each of the test regions comprises an array of generic anchor molecules. The anchors are associated with bifunctional linker molecules, each containing a portion which is specific for at least one of the anchors and a portion which is a probe specific for a target of interest. The resulting array of probes is used to analyze the presence or test the activity of one or more target molecules which specifically interact with the probes.

### SUMMARY OF THE INVENTION

Exemplary embodiments of the invention include a method of efficiently producing a microarray with a high spot density and with a long probe immobilized thereon.

Additional aspects will be set forth in part in the description which follows and, in part, will be apparent from the description, or may be learned by practice of the presented embodiments.

The invention refers to a method of producing a microarray having an immobilized double-stranded nucleic acid probe including a double-stranded region and a single-stranded region wherein the single-stranded region acts as a probe that may hybridize with a target nucleic acid, the method including: synthesizing a nucleic acid on a spot region on a surface of a solid substrate by using photolithography to immobilize a first single-stranded nucleic acid in the spot region; and hybridizing the first single-stranded nucleic acid with a second single-stranded nucleic acid including a first region that is complementary to the first single-stranded nucleic acid and a second region that is not complementary to the first single-stranded nucleic acid to convert the first single-stranded nucleic acid to the double-stranded nucleic acid probe including a double-stranded region and a single-stranded region, wherein the second region is positioned upstream from the first region when a 5' terminal of the first single-stranded nucleic acid is attached to the surface of the substrate, and the second region is positioned downstream from the first region when a 3' terminal of the first single-stranded nucleic acid is attached to the surface of the substrate; and reacting the double-stranded nucleic acid probe with a material that specifically binds to a double-stranded region of nucleic acid, thereby enhancing hybridization binding between the second single-stranded nucleic acid and the first single-stranded nucleic acid characterized in that the material that specifically binds a double-stranded region of nucleic acid comprises a double-strand specific-binding intercalator and in that the proximal terminal of the second single-stranded nucleic acid on the surface of the substrate is immobilized on the substrate by a covalent or non-covalent bond.
The term "a" has to be understood as at least one of and comprises only one as well as a plurality of entities. A preferred embodiment of the invention may include a method of producing a microarray having an immobilized double-stranded nucleic acid probe including a double-stranded region and a single-stranded region, the method including: synthesizing nucleic acids on a plurality of spot regions on the surface of the solid substrate by using photolithography to immobilize a plurality of first single-stranded nucleic acids on the plurality of spot regions; and hybridizing a plurality, preferably each of the plurality of first single-stranded nucleic acids with a second single-stranded nucleic acid including a first region complementary to the plurality of first single-stranded nucleic acids and a second region that is not complementary to the plurality of first single-stranded nucleic acids to convert the plurality of first single-stranded nucleic acids to double-stranded nucleic acid probes including a double-stranded region and a single-stranded region, wherein the second region is positioned upstream from the first region when a 5' terminal of the first single-stranded nucleic acid is attached to the surface of the substrate, and the second region is positioned downstream from the first region when a 3' terminal of the first single-stranded nucleic acid is attached to the surface of the substrate. The first single-stranded nucleic acids synthesized in the different spot regions may have the same or different sequences.

### BRIEF DESCRIPTION OF THE DRAWINGS

FIGS. 1A and 1B are diagrams illustrating a method of producing a single-stranded microarray in a method of producing a microarray having an immobilized double-stranded nucleic acid probe, according to embodiments of the invention.

FIG. 2 is a diagram illustrating a method of converting the single-stranded microarray prepared in FIG. 1 to a double-stranded microarray.

### DETAILED DESCRIPTION OF EXEMPLARY EMBODIMENTS

Reference will now be made in detail to embodiments, examples of which are illustrated in the accompanying drawings, wherein like reference numerals refer to the like elements throughout. In this regard, the present embodiments may have different forms and should not be construed as being limited to the descriptions set forth herein. Accordingly, the embodiments are merely described below, by referring to the figures, to explain aspects of the present description.

A method according to the invention of producing a microarray having an immobilized double-stranded nucleic acid probe including a double-stranded region and a single-stranded region according to claim 1 includes (a) synthesizing nucleic acids on a plurality of spot regions on a surface of a solid substrate by using photolithography to immobilize a plurality of first single-stranded nucleic acids on the spot regions.

The term "photolithography" includes selectively exposing a photosensitive surface to a light beam to form a pattern of light exposed areas and light nonexposed areas on the surface and reacting a molecule with the light exposed area to couple the molecule to the light exposed area. The photosensitive surface may include a chemically reactive group protected by a photoremovable group, and when the photosensitive surface is exposed to a light, the surface may be become reactive due to the removal of the photoremovable group. The reactant molecule may include an activated nucleoside, an activated nucleotide, an activated oligonucleotide or an activated polynucleotide protected with a photoremovable group at 3' hydroxyl or 5' hydroxyl group. Selectively exposing a photosensitive surface to a light beam may be conducted by placing a physical photomask in the path of light used for photoactivation. It may also be conducted without using a physical photomask, for examples by using a maskless photolithography system. The maskless photolithography systems is known to one of ordinary skill in the art and may include a system using an off-axis light source coupled with a digital micromirror array.

The operation of "synthesizing nucleic acids on the substrate by using photolithography" means that only certain regions on the substrate are irradiated by light, thereby introducing or producing an active reaction group on only the certain regions on the substrate, and the active reaction group is reacted with an activated molecules such as, nucleic acid monomer, oligomer or polymer, to extend a nucleotide of a nucleic acid on the substrate. It may include synthesizing a single stranded polynucleotide by extending one nucleotide by one nucleotide on the substrate. Light-directed synthesis methods of nucleic acids on a substrate are well known in the art. For example, these methods are disclosed in US Patent Nos. 5,143,854, 5,744,305, and 5,908,926, the disclosures of which are herein incorporated by reference in their entireties. The double-stranded region of the double-stranded nucleic acid probe is positioned on the surface of a substrate, that is, proximal to the surface of a substrate, and the single-stranded region thereof is positioned distal from the surface of a substrate. The double-stranded region may include the first single stranded nucleic acid hybridized to a part of the second single-stranded nucleic acid, and the single-stranded region may include only the second single-stranded nucleic acid. The second single-stranded nucleic acids may include two regions that is, the region that forms a double stranded nucleic acid by hybridizing with the first single-stranded nucleic acid and the region that does not take part in the hybridization with the first single-stranded nucleic acid and thus, may remain as a single stranded form. The double-stranded nucleic acid probe may be a partial double stranded nucleic acid wherein the double stranded region is proximal to the substrate and the single stranded region is distal from the substrate. The single stranded region of the double-stranded nucleic acid probe may hybridize with a target nucleic acid having a complementary sequence, and thus acting as a probe.

In the operation of "synthesizing nucleic acids on the substrate by using photolithography," a plurality of similar and/or different nucleic acid sequences may be simultaneously synthesized in parallel or consecutively on a plurality of predefined regions (hereinafter, referred to as "spots") on a substrate.

The term "spot" used herein refers to a certain region on a surface, which is activated, previously activated, or desired to be activated for formation of a polymer, that is, a "predefined region." The spot may have a convenient shape, for example, a circular, tetragonal, oval, or wedge-like shape.

The term "substrate" used herein refers to a material with a solid or semisolid surface. Although a surface of a substrate may be substantially flat, at least a portion of a surface of a substrate may be physically isolated as a synthesis region with respect to other nucleic acids synthesis regions by a well, a heightened region, or an etched trench, etc.

The "light" used herein may be gamma-rays, X-rays, ultraviolet rays, visible rays, or infrared rays, and may be appropriately selected according to the type of a photoremovable protecting group used.

The operation (a) includes selectively irradiating a surface of a substrate with light through a patterned mask, selectively activating certain regions on the surface of the substrate, and reacting the activated regions with nucleic acid monomers, thereby extending nucleotides on the surface of the substrate. The selective activation of the certain regions on the surface of the substrate may be performed by light irradiation, when an active group is protected by a photoremovable protecting group on the surface of the substrate. In addition, the activation process may be performed by a chemical material for removing the photoremovable protecting group.

The operation (a) may include irradiating the surface of the substrate to which the photoremovable protecting group is attached with light through a mask to selectively remove the protecting group from the surface in the illuminated regions, thereby forming a pattern of reactive regions and light protected regions, and reacting the surface of the reactive regions with activated nucleic acid monomers having the photoremovable protecting group, thereby extending the nucleic acid monomers to the reactive regions on the substrate. The photoremovable protecting group on the substrate may be the same or different to the photoremovable protecting group of the activated nucleic acid monomer. The photoremovable protecting group may be removed from a surface of an irradiated region, which is irradiated by light, and thus the active reaction group is exposed. Thus, light-exposed active reactive group on the surface of the irradiated region may be reacted with the activated nucleic acid monomer. The nucleic acid monomer may be a nucleoside or a nucleotide or an oligonucleotide or polynucleotide.

The photoremovable protecting group attached to the surface of the substrate may be attached to the substrate through a linker. The linker may have an active group at a terminal of the linker distal from the surface of the substrate that is protected by a photoremovable protecting group. Any linker that may provide a reactive group protected by the photoremovable protecting group may be used. For example, the linker may be polyethyleneglycols, or nucleic acid monomers, such as nucleosides and nucleotides.

The term "protecting group" used herein refers to a group that is chemically bound to a monomer unit and may be removed by a selective exposure to an activator such electromagnetic radiation. The term "photoremovable protecting group" used herein refers to a protecting group that may be removed by light. Photoremovable protecting groups are well known to those of ordinary skill in the art. Examples of the photoremovable protecting group may include, but are not limited to, 6-nitroveratryloxycarbonyl (NVOC), 6-nitropiperonyl (NP), 6-nitropiperonyl oxycarbonyl (NPOC), 6-nitroveratryl (NV), methyl-6-nitroveratryl (MeNV), methyl-6-nitroveratryloxycarbonyl (MeNVOC), methyl-6-nitropiperonyl (MeNP), and methyl-6-nitropiperonyloxycarbonyl (MeNPOC).

As shown in Formula 1 below, the photoremovable protecting group may be attached to an activated nucleotide on the 5'-hydroxyl group: 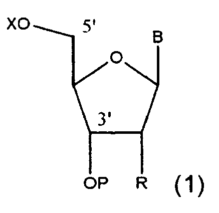 wherein B is a base bound to a sugar ring, R is a hydrogen atom when the sugar is deoxyribose, or R is a hydroxyl group when the sugar is ribose, P is an activated phosphoric acid group, and X is a photoremovable protecting group. The photoremovable protecting group X may be, as described above, NV, NP, MeNV, or MeNP. The activated phosphoric acid group P may be a reactive derivative with high coupling efficiency, such as phosphate triester, or phosphoramidite. Reaction conditions and other activated phosphoric acid groups are well known (see for example, US Patent Nos. 5,143,854, 5,744,305, and 5,908,926). In addition, a reactive group of the base is screened by a protecting group, and the screening and non-screening of the base are known in the art. The base may be uracil, adenine, guanine, thymine, cytosine, or inosine.

In addition, as shown in Formula 2 below, the photoremovable protecting group may be attached to an activated nucleotide on the 3'-hydroxyl group. The activated nucleotide in which the 3'-hydroxyl group is screened by the photoremovable protecting group may be a compound represented by Formula 2 below: 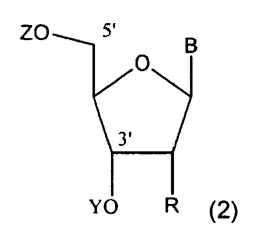 wherein B is a base in which an active group bound to a sugar ring is screened, R is a hydrogen atom when the sugar is deoxyribose, or R is a hydroxyl group when the sugar is ribose, Y includes a benzoinyl carbonate, such as dialkoxybenzoinyl carbonate, di(C₁-C₄)alkoxybenzoinyl carbonate, or 3',5' or 2',3'-dimethoxybenzoinyl carbonate, and Z is phosphoramidite. The base may be uracil, adenine, guanine, thymine, cytosine, or inosine.

The linker may be selected from the group consisting of nucleosides and nucleotides, wherein a 3'-terminal of one of the nucleosides and nucleotides is attached to a substrate and a 5'-terminal of one of the nucleosides and nucleotides is attached to the photoremovable protecting group, or a 5'-terminal is attached to the substrate and a 3'-terminal is attached to the photoremovable protecting group. In addition, the nucleotides include polynucleotides that are synthesized in a previous process, or are foreign-introduced.

The formation of the pattern and the extension of the nucleic acid monomers may be repeatedly performed. In each repetition cycle, the activated monomer reacts in the spot regions of the substrate that are patterned into reactive regions by removing the photoremovable protecting group, thereby binding to and extending the nucleic acid synthesized in this spot region so far. In this case, a general washing process may be performed between the formation of the pattern and the extension of the nucleic acid monomers and/or between the extension of the nucleic acid monomers and the formation of the pattern. In addition, in the formation of the pattern, patterns that are the same as or different from each other may be formed according to the type of nucleotide to be introduced in each spot. In this process, one mask may be used for one nucleotide. Thus, when a 25 mer nucleic acid is synthesized, up to 100 masks may be used. Therefore, 100 cycles of mask preparation, light irradiation, detachment of the photoremovable protecting group, addition of the activated monomer having a photoremovable protecting group, and the coupling reaction are used.

The first single-stranded nucleic acid or the plurality of the first single-stranded nucleic acids may be selected from the group consisting of DNA, RNA, PNA, and hybrid molecules thereof. When the first single-stranded nucleic acid is PNA, a dissociation constant of a hybridization product is low, and thus the double-stranded region is stable. The type of nucleic acid of the plurality of the first single-stranded nucleic acids may be the same or different for first single-stranded nucleic acids in different spot regions.

The length of the first single-stranded nucleic acid or the plurality of the first single-stranded nucleic acids may be a length that may provide a binding force coupling the first single-stranded nucleic acid to the second single-stranded nucleic acid, obtained by hybridizing the first single-stranded nucleic acid with a first region of the second single-stranded nucleic acid. For example, the length may be about 4 nt to about 25 nt, about 5 nt to about 10 nt, or about 10 nt to about 25 nt. The length of the plurality of the first single-stranded nucleic acids may be the same or different for first single-stranded nucleic acids in different spot regions.

The density of spots on the substrate on which a first single-stranded nucleic acid is immobilized may be 150,000 spots/cm² or greater. Light-directed synthesis methods of nucleic acids use a selective activation of a surface by using a patterned light, for example by using a photomask in the path of a light, and thus spots may be arranged with high density. However, a large amount of mask and many steps are used, and thus there is a limitation on a length to be synthesized.

The method of producing a microarray having an immobilized double-stranded nucleic acid probe including a double-stranded region and a single-stranded region further includes storing a sequence of the first single-stranded nucleic acid and a position on a substrate of a spot on which the sequence thereof is immobilized. Preferably, the sequences of a plurality of, preferably all of the first single-stranded nucleic acids is stored in combination with and depending on the position of their corresponding spot region. The sequence of the first single-stranded nucleic acid is known, and the position on the substrate thereof is identified, and thus the position where the second single-stranded nucleic acid is hybridized may be confirmed. From this, a position of a double-stranded nucleic acid probe may be identified, and hybridization results obtained as a result of microarray analysis may be analyzed on each spot.

According to an embodiment of the invention, the operation (a) may further include: (a-1) selectively irradiating with light a first region of a spot region on a surface of a substrate and not irradiating with light a second region of the spot region on the surface, thereby forming a pattern of bright, light-exposed and dark, non-light exposed regions, wherein the surface of the substrate has a photoremovable protecting group; (a-2) contacting the first region and the second region of the spot region of the surface of the substrate with a first nucleotide to link the first nucleotide to a polynucleotide of the first region and not to link the first nucleotide to the second region, wherein the first nucleotide has a photoremovable protecting group; (a-3) selectively irradiating with electromagnetic radiation at least one portion selected from the group consisting of at least one portion of the first region of the surface of the substrate and at least one portion of the second region of the surface of the substrate to remove the photoremovable protecting group therefrom; and (a-4) contacting the first region and the second region of the surface of the substrate with a second nucleotide to link the second nucleotide to a polynucleotide of the portion from which the photoremovable protecting group is removed.

According to an embodiment of the invention, the operation (a) may include: (a-1) attaching to a support a nucleoside including a photoremovable 3' hydroxyl protecting group, wherein the attachment is via a 5' hydroxyl of the nucleoside; (a-2) irradiating the obtained support-bound nucleoside so that the photoremovable protecting group is removed and the 3' hydroxyl group is thereby freed and ready for reaction; and (a-3) contacting the support-bound nucleoside with a nucleotide including a 5 ' phosphoramidite to react the 5 ' phosphoramidite with the free 3' hydroxyl so that a dinucleotide is formed.

In addition, the method of producing a microarray having an immobilized double-stranded nucleic acid probe including a double-stranded region and a single-stranded region includes (b) hybridizing each of the first single-stranded nucleic acids with a second single-stranded nucleic acid including a first region complementary to the first single-stranded nucleic acid and a second region that is not complementary to the first single-stranded nucleic acid to convert the first single-stranded nucleic acid to a double-stranded nucleic acid probe including a double-stranded region and a single-stranded region. The second region is positioned upstream from the first region when a 5' terminal of the first single-stranded nucleic acid is attached to the surface of the substrate, and the second region is positioned downstream from the first region when a 3' terminal of the first single-stranded nucleic acid is attached to the surface of the substrate. The term "upstream" or "downstream" refers to relative position from a reference position in the nucleic acid. For example, when a sequence is positioned in the 5' hydroxyl side from a reference position, the sequence is positioned in the upstream position and when a sequence is positioned in the 3' hydroxyl side from a reference position, the sequence is positioned in the downstream position.

In operation (b), the hybridization may be performed under conditions that are known in the art, for example, in a common hybridization buffer at 4 °C for 14 hours.

The first region of the second single-stranded nucleic acid is complementary to the first single-stranded nucleic acid, and thus the second single-stranded nucleic acid may be immobilized on the first single-stranded nucleic acid due to hybridization. The term "complementary" used herein means that 85% or greater, 90% or greater, 95% or greater, or 100% of sequences between nucleic acids are complementary to each other. In addition, the second region of the second single-stranded nucleic acid includes a sequence that is non-complementary to the first single-stranded nucleic acid. The term "non-complementary" used herein means that the second region is not substantially complementary to the first single-stranded nucleic acid, thus not interfering with hybridization between the first region and the first single-stranded nucleic acid. The second region may include a contiguous sequence of 5 nucleotides or less, 4 nucleotides or less, 3 nucleotides or less, or 2 nucleotides or less, which are complementary to the first single-stranded nucleic acid. The first region of the second single-stranded nucleic acid may be complementary to the first single-stranded nucleic acid with 100% identity and the second region of the second single-stranded nucleic acid may not contain a sequence that is complementary to the first single-stranded nucleic acid.

The second single-stranded nucleic acid may be synthesized in a medium that is different from a solid substrate in which the first single-stranded nucleic acid is synthesized or derived from natural nucleic acids. The synthesis of the second single-stranded nucleic acid in the different medium is well known in the art.

For example, synthesis of nucleic acids generally involves coupling an activated phosphoric acid derivative on a 3' hydroxyl of a nucleotide with a 5' hydroxyl of an oligomer bound to a solid substrate. The coupling reaction may be induced using two chemical methods: phosphate triester and phosphoramidite methods (Gait, "Oligonucleotide Synthesis": A practical Approach", 1984, IRL, Press, London).

The second single-stranded nucleic acid may be synthesized in a solid medium without using a photolithography process. In addition, the second single-stranded nucleic acid may be cDNA or be derived from a nucleic acid amplified by a nucleic acid amplification method, for example, PCR. Since the second single-stranded nucleic acid may not be synthesized in parallel at a plurality of positions, for example, through a mask, a relatively long nucleic acid may be synthesized with high efficiency. The second single-stranded nucleic acid may have a length of 50 nt or more, about 50 nt to about 3000 nt, or about 50 nt to about 1000 nt. In addition, the second single-stranded nucleic acid may be synthesized using a cell, or obtained by digesting natural nucleic acids with a restriction enzyme. The second region of the second single-stranded nucleic acid may have a length of 25 nt or more, about 25 nt to about 2975 nt, or about 25 nt to about 975 nt.

The method may further include, in particular in operation (b) the step of setting the position of the double-stranded nucleic acid probe on the substrate to correspond to a position of the first single-stranded nucleic acid that is complementary to the first region of the second single-stranded nucleic acid, and storing the position of the double-stranded nucleic acid probe. The storing process may be performed in a computer readable medium. Thus, the position of the probe and/or hybridization results between a target nucleic acid and the probe may be confirmed and analyzed using a computer. That is, the sequence of the first single-stranded nucleic acid or the first region of the second single-stranded nucleic acid may be used as accessible information that may confirm the position of the probe or the analysis results of the hybridization. Preferably, the position of a plurality of, more preferred each of the double-stranded nucleic acid probe is set on the substrate to correspond to the position of the plurality of first single-stranded nucleic acids that are complementary to the first region of the second single-stranded nucleic acid, which converts the first single-stranded nucleic acid to the double-stranded nucleic acid probe, and storing the position of the double-stranded nucleic acid probe

One of the first single-stranded nucleic acid and the first region of the second single-stranded nucleic acid may be PNA, and the other thereof may be DNA or RNA. Hybridization intensity between PNA and DNA or RNA is relatively stronger than hybridization intensity between DNAs, RNAs, or DNA and RNA, and thus the second single-stranded nucleic acid may be stably immobilized on the first single-stranded nucleic acid.

The method of producing a microarray having an immobilized double-stranded nucleic acid probe including a double-stranded region and a single-stranded region may further include (c) reacting the double-stranded nucleic acid probe with a material that specifically binds to the double-stranded region of the double-stranded nucleic acid probe, thereby enhancing hybridization binding between the second single-stranded nucleic acid and the first single-stranded nucleic acid.

The material that specifically binds a double-stranded region of nucleic acid may be a double-strand specific-binding intercalator. The double-strand specific-binding intercalator may be SYBR Green I. The double-strand specific-binding intercalator better stabilizes a double bond, and thus decreases the possibility of dissociating the double-stranded region of the double-stranded nucleic acid probe in the process of analyzing the double-stranded nucleic acid probe. In addition, the proximal terminal of the second single-stranded nucleic acid on the surface of the substrate is immobilized the sub-strate by a covalent bond or non-covalent bond.

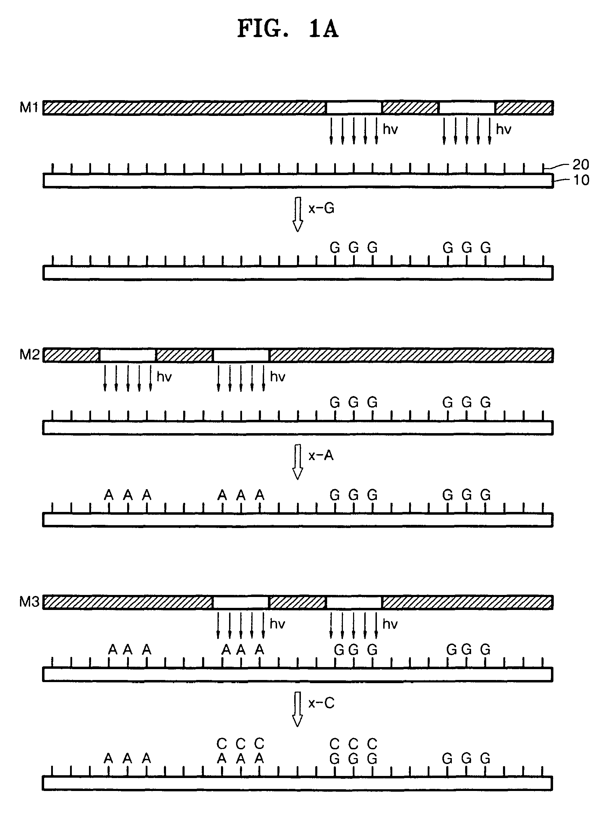

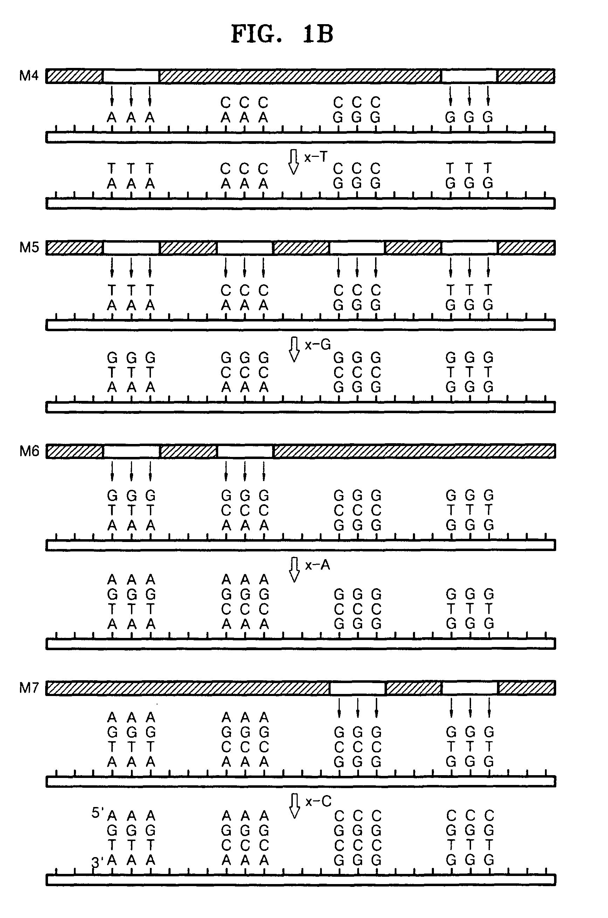

FIGS. 1A and 1B are diagrams illustrating a method of producing a single-stranded nucleic acid microarray having an immobilized double-stranded nucleic acid probe, according to embodiments of the invention. In FIGS. 1A and 1B, M1 through M7 denote masks, 10 denotes a substrate, and 20 denotes a photoremovable protecting group immobilized on a surface of the substrate 10. Referring to FIGS. 1A and 1B, in the masks M1 through M7, bright portions are regions through which light is transmitted, and hatched portions are regions through which light is not transmitted. The photoremovable protecting group 20 may be linked to a linker, a nucleic acid monomer, or an oligomer. In addition, the x in x-G, x-A, x-C, and x-T denotes a photoremovable protecting group where G, A, C, and T are the bases guanine, adenine, cytosine, and thymine, respectively. First, referring to FIG. 1A, when the substrate 10 is irradiated with light through the mask M1, the photoremovable protecting group 20 immobilized on the substrate 10 is removed from a predefined region that is irradiated by the light, and as a result, an active group is exposed. Then, the surface of the substrate 10 with the exposed active group is reacted with first activated nucleic acid monomers having a photoremovable protecting group to attach the first activated nucleic acid monomers to the surface of the substrate 10. Unreacted first activated nucleic acid monomers are removed by washing, and then predefined regions on the surface of the substrate 10 are irradiated with light through the mask M2, and the surface of the substrate 10 is reacted with second activated nucleic acid monomers having a photoremovable protecting group, thereby linking the second activated nucleic acid monomers to the first activated nucleic acid monomers. By repeatedly performing such processes using the masks M3 through M7, a single-stranded nucleic acid microarray on which four different single-stranded nucleic acid probes are respectively immobilized on four spot regions is prepared. In the first and second activated nucleic acid monomers having a photoremovable protecting group, the photoremovable protecting group may be, as shown in Formula 1 or 2 above, attached to the activated nucleotide on the 5'-hydroxyl or the 3'-hydroxyl. Referring to FIGS. 1A and 1B, the photoremovable protecting group is attached to the activated nucleotide on the 5'-hydroxyl. As shown in FIGS. 1A and 1B, when the light-directed synthesis of the single-stranded nucleic acids on a plurality of predefined regions is performed by activating the substrate 10 through the masks M1 through M7, the distance between the predefined regions may be narrowed. That is, the density of predetermined regions (spots) may be increased in particular compared to the interval between adjacent spots in a microarray produced by the spotting method. However, the plurality of masks prepared in this case complicates the manufacturing processes.

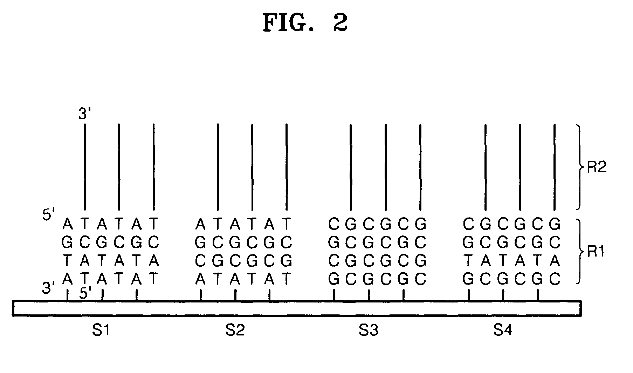

FIG. 2 is a diagram illustrating a method of converting the single-stranded microarray prepared in FIG. 1 to a double-stranded microarray, according to an embodiment of the invention. In FIG. 2, S1 through S4 denote spot regions on a substrate. Single-stranded nucleic acids of different sequences are respectively immobilized on the spot regions S1 through S4. In addition, in FIG. 2, R1 and R2 denote a double-stranded region and a single-stranded region of a double-stranded nucleic acid probe, respectively. Referring to FIG. 2, a first single-stranded nucleic acid of the double-stranded nucleic acid probe is synthesized on the substrate in the direction of 3' -> 5', and thus a second single-stranded nucleic acid including a complementary sequence to the first single-stranded nucleic acid is arranged in the direction of 5' -> 3' from the substrate. However, an opposite case is also possible, in which the first single-stranded nucleic acid is synthesized on the substrate in the direction of 5' -> 3', and the second single-stranded nucleic acid including a complementary sequence to the first single-stranded nucleic acid is arranged in the direction of 3' -> 5' from the substrate. The second single-stranded nucleic acid may be obtained without using a mask by known solid-phase nucleic acid synthesis or by digesting natural nucleic acids with a restriction enzyme, and thus the second single-stranded nucleic acid may be relatively long. For example, the single-stranded region R2 may have a length of about 30 nt to about 1000 nt. In addition, although not illustrated in FIG. 2, the double-stranded region R1 may be treated with a material that specifically binds the double-stranded region R1 to enhance hybridization binding. For example, the material may be a double strand specific-binding intercalator compound, such as SYBR Green I. In addition, the proximal terminal of the second single-stranded nucleic acid on the substrate may be immobilized on the first single-stranded nucleic acid or the substrate by a covalent or non-covalent bond.

As described above, according to the above exemplary embodiments of the invention, by using a method of preparing a microarray on which a double-stranded nucleic acid probe including a double-stranded region and a terminal single-stranded region is immobilized, a microarray with high spot density and on which a long double-stranded nucleic acid probe is immobilized may be efficiently prepared.

It should be understood that the exemplary embodiments described herein should be considered in a descriptive sense only and not for purposes of limitation. Descriptions of features or aspects within each embodiment of the invention should typically be considered as available for other similar features or aspects in other embodiments of the invention.

## Claims

1. A method of producing a microarray having an immobilized double-stranded nucleic acid probe comprising a double-stranded region and a single-stranded region wherein the single-stranded region acts as a probe that may hybridize with a target nucleic acid, the method comprising:
synthesizing a nucleic acid on a spot region on a surface of a solid substrate using photolithography to immobilize a first single-stranded nucleic acid in the spot region;
hybridizing the first single-stranded nucleic acid with a second single-stranded nucleic acid comprising a first region that is complementary to the first single-stranded nucleic acid and a second region that is not complementary to the first single-stranded nucleic acid to convert the first single-stranded nucleic acid to the double-stranded nucleic acid probe comprising a double-stranded region and a single-stranded region, wherein the second region is positioned upstream from the first region when a 5' terminal of the first single-stranded nucleic acid is attached to the surface of the substrate, and the second region is positioned downstream from the first region when a 3' terminal of the first single-stranded nucleic acid is attached to the surface of the substrate; and
reacting the double-stranded nucleic acid probe with a material that specifically binds to a double-stranded region of nucleic acid, thereby enhancing hybridization binding between the second single-stranded nucleic acid and the first single-stranded nucleic acid, **characterized in that** the material that specifically binds a double-stranded region of nucleic acid comprises a double-strand specific-binding intercalator and **in that** the proximal terminal of the second single-stranded nucleic acid on the surface of the substrate is immobilized on the substrate by a covalent or non-covalent bond.

2. The method of claim 1, wherein the synthesizing of the nucleic acid comprises irradiating the surface of the substrate to which a photoremovable protecting group is attached with light through a mask to selectively remove the protecting group from the surface, thereby forming a pattern of reactive regions and light protected regions, and reacting a reactive region with an activated nucleic acid monomer having a photoremovable protecting group, thereby binding the nucleic acid monomer to the reactive regions on the substrate.

3. The method of claim 2, wherein the photoremovable protecting group attached to the substrate is attached to the substrate through a linker having an active group that is protected by the photoremovable protecting group and that is arranged at the distal terminal facing away from the surface of the substrate.

4. The method of claim 3, wherein the linker is selected from the group consisting of a nucleoside and a nucleotide of which a 3'-terminal is attached to the substrate and a 5'-terminal is attached to the photoremovable protecting group, or of which a 5'-terminal is attached to the substrate and a 3'-terminal is attached to the photoremovable protecting group.

5. The method of any one of claims 2 to 4, wherein the synthesizing and reacting are repeatedly performed.

6. The method of any one of claims 1 to 5, wherein the first single-stranded nucleic acid is selected from the group consisting of DNA, RNA, PNA, and a hybrid molecule thereof.

7. The method of any one of claim 1 to 6, wherein the first single-stranded nucleic acid has a length of about 4 nt to about 25 nt.

8. The method of any one of claims 1 to 7, further comprising storing the sequence of the first single-stranded nucleic acid and the corresponding position of the spot region on the substrate in which the sequence is immobilized.

9. The method of any one of claims 1 to 8, wherein the second single-stranded nucleic acid is synthesized in a medium different from the solid substrate in which the first single-stranded nucleic acid is synthesized.

10. The method of claim 9, wherein the second single-stranded nucleic acid comprises cDNA or DNA derived from a nucleic acid obtained by nucleic acid amplification.

11. The method of claim 9 or 10, wherein the second single-stranded nucleic acid has a length of 50 nt or greater, preferably a length of about 50 nt to about 1000 nt.

12. The method of any of claims 1 to 11, wherein the hybridizing further comprises setting the position of the double-stranded nucleic acid probe on the substrate to correspond to the position of the first single-stranded nucleic acid that is complementary to the first region of a second single-stranded nucleic acid, which second single-stranded nucleic acid converts the first single-stranded nucleic acid to the double-stranded nucleic acid probe, and storing the position of the double-stranded nucleic acid probe.

13. The method of any one of claims 1 to 12, wherein one of the first single-stranded nucleic acid and the complementary first region of the second single-stranded nucleic acid is PNA, and the other thereof is DNA or RNA.

## Patentansprüche

1. Verfahren zum Herstellen eines Microarrays, der eine immobilisierte doppelsträngige Nucleinsäuresonde aufweist, welche einen doppelsträngigen Bereich und einen einzelsträngigen Bereich umfasst, wobei der einzelsträngige Bereich als Sonde fungiert, die mit einer Ziel-Nucleinsäure hybridisieren kann, wobei das Verfahren umfasst:
Synthetisieren einer Nucleinsäure auf einem Spotbereich auf einer Oberfläche eines festen Substrats unter Verwendung von Photolithographie, um eine erste einzelsträngige Nucleinsäure in dem Spotbereich zu immobilisieren;
Hybridisieren der ersten einzelsträngigen Nucleinsäure mit einer zweiten einzelsträngigen Nucleinsäure, welche einen ersten Bereich, der zu der ersten einzelsträngigen Nucleinsäure komplementär ist, und einen zweiten Bereich, der zu der ersten einzelsträngigen Nucleinsäure nicht komplementär ist, umfasst, um die erste einzelsträngige Nucleinsäure in die doppelsträngige Nucleinsäuresonde umzuwandeln, welche einen doppelsträngigen Bereich und einen einzelsträngigen Bereich umfasst, wobei sich der zweite Bereich stromaufwärts des ersten Bereichs befindet, wenn ein 5'-Terminus der ersten einzelsträngigen Nucleinsäure an der Oberfläche des Substrats befestigt ist, und sich der zweite Bereich stromabwärts des ersten Bereichs befindet, wenn ein 3'-Terminus der ersten einzelsträngigen Nucleinsäure an der Oberfläche des Substrats befestigt ist, und
Umsetzen der doppelsträngigen Nucleinsäuresonde mit einem Material, das spezifisch an einen doppelsträngigen Nucleinsäurebereich bindet, wodurch die Hybridisierungsbindung zwischen der zweiten einzelsträngigen Nucleinsäure und der ersten einzelsträngigen Nucleinsäure verstärkt wird, **dadurch gekennzeichnet, dass** das Material, das spezifisch an einen doppelsträngigen Nucleinsäurebereich bindet, einen doppelstrangspezifischen Bindungsinterkalator umfasst und **dadurch**, dass der proximale Terminus der zweiten einzelsträngigen Nucleinsäure auf der Oberfläche des Substrats durch eine kovalente oder nicht kovalente Bindung auf dem Substrat immobilisiert ist.

2. Verfahren nach Anspruch 1, wobei das Synthetisieren der Nucleinsäure das Bestrahlen der Oberfläche des Substrats, an welchem eine photolabile Schutzgruppe befestigt ist, mit Licht durch eine Maske umfasst, um die Schutzgruppe selektiv von der Oberfläche zu entfernen, wodurch ein Muster von reaktionsfähigen Bereichen und lichtgeschützten Bereichen gebildet wird, und Umsetzen eines reaktionsfähigen Bereichs mit einem aktivierten Nucleinsäuremonomer, welches eine photolabile Schutzgruppe aufweist, wodurch das Nucleinsäuremonomer an die reaktionsfähigen Bereiche auf dem Substrat gebunden wird.

3. Verfahren nach Anspruch 2, wobei die an dem Substrat befestigte photolabile Schutzgruppe an dem Substrat mittels eines Linkers befestigt ist, der eine aktive Gruppe aufweist, die durch die photolabile Schutzgruppe geschützt ist und die an dem distalen Terminus angebracht ist, der von der Oberfläche des Substrats weg zeigt.

4. Verfahren nach Anspruch 3, wobei der Linker aus der Gruppe ausgewählt ist, bestehend aus einem Nucleosid und einem Nucleotid, von dem ein 3'-Terminus an dem Substrat befestigt ist und von dem ein 5'-Terminus an der photolabilen Schutzgruppe befestigt ist, oder von dem ein 5'-Terminus an dem Substrat befestigt ist und ein 3'-Terminus an der photolabilen Schutzgruppe befestigt ist.

5. Verfahren nach einem der Ansprüche 2 bis 4, wobei das Synthetisieren und Umsetzen wiederholt durchgeführt werden.

6. Verfahren nach einem der Ansprüche 1 bis 5, wobei die erste einzelsträngige Nucleinsäure aus der Gruppe ausgewählt ist, bestehend aus DNA, RNA, PNA und einem Hybridmolekül davon.

7. Verfahren nach einem der Ansprüche 1 bis 6, wobei die erste einzelsträngige Nucleinsäure eine Länge von etwa 4 nt bis etwa 25 nt aufweist.

8. Verfahren nach einem der Ansprüche 1 bis 7, ferner umfassend das Speichern der Sequenz der ersten einzelsträngigen Nucleinsäure und der entsprechenden Position des Spotbereichs auf dem Substrat, in dem die Sequenz immobilisiert ist.

9. Verfahren nach einem der Ansprüche 1 bis 8, wobei die zweite einzelsträngige Nucleinsäure in einem anderen Medium als dem festen Substrat synthetisiert wird, in dem die erste einzelsträngige Nucleinsäure synthetisiert wird.

10. Verfahren nach Anspruch 9, wobei die zweite einzelsträngige Nucleinsäure cDNA oder DNA umfasst, die von einer durch Nucleinsäureamplifikation erhaltenen Nucleinsäure abgeleitet ist.

11. Verfahren nach Anspruch 9 oder 10, wobei die zweite einzelsträngige Nucleinsäure eine Länge von 50 nt oder mehr, vorzugsweise eine Länge von etwa 50 nt bis etwa 1000 nt, aufweist.

12. Verfahren nach einem der Ansprüche 1 bis 11, wobei das Hybridisieren ferner Setzen der Position der doppelsträngigen Nucleinsäuresonde auf dem Substrat, um der Position der ersten einzelsträngigen Nucleinsäure zu entsprechen, die zu dem ersten Bereich einer zweiten einzelsträngigen Nucleinsäure komplementär ist, wobei die zweite einzelsträngige Nucleinsäure die erste einzelsträngige Nucleinsäure in die doppelsträngige Nucleinsäuresonde umwandelt, und Speichern der Position der doppelsträngigen Nucleinsäuresonde umfasst.

13. Verfahren nach einem der Ansprüche 1 bis 12, wobei es sich bei einer aus der ersten einzelsträngigen Nucleinsäure und dem komplementären ersten Bereich der zweiten einzelsträngigen Nucleinsäure um PNA handelt und es sich bei der anderen davon um DNA oder RNA handelt.

## Revendications

1. Procédé de fabrication d'un micro réseau comportant une sonde d'acide nucléique à double brin immobilisé comprenant une région à double brin et une région à simple brin, dans lequel la région à simple brin agit comme une sonde qui peut être hybridée avec un acide nucléique cible, le procédé comprenant :
la synthèse d'un acide nucléique sur une région ponctuelle située sur une surface d'un substrat solide en utilisant la photolithographie pour immobiliser un premier acide nucléique à simple brin dans la région ponctuelle,
l'hybridation du première acide nucléique à simple brin avec un second acide nucléique à simple brin comprenant une première région qui est complémentaire du premier acide nucléique à simple brin, ainsi qu'une seconde région qui n'est pas complémentaire du premier acide nucléique à simple brin, pour convertir le premier acide nucléique à simple brin en la sonde d'acide nucléique à double brin comprenant une région à double brin et une région à simple brin, la seconde région étant positionnée en amont de la première région lorsqu'une terminaison 5' du premier acide nucléique à simple brin est fixée à la surface du substrat, et la seconde région étant positionnée en aval de la première région lorsqu'une terminaison 3' du premier acide nucléique à simple brin est fixée à la surface du substrat, et
la réaction de la sonde d'acide nucléique à double brin avec une matière qui se lie spécifiquement à une région à double brin d'acide nucléique, ce qui améliore de ce fait la liaison d'hybridation entre le second acide nucléique à simple brin et le premier acide nucléique à simple brin, **caractérisé en ce que** la matière qui se lie spécifiquement à une région à double brin d'acide nucléique comprend un agent d'intercalation à double brin spécifique à la liaison et **en ce que** la terminaison proximale du second acide nucléique à simple brin sur la surface du substrat est immobilisée sur le substrat par une liaison covalente ou non covalente.

2. Procédé selon la revendication 1, dans lequel la synthèse de l'acide nucléique comprend l'illumination avec une lumière de la surface du substrat auquel un groupe de protection photo amovible est fixé au travers d'un masque afin de supprimer sélectivement le groupe de protection de la surface, formant ainsi un motif de régions réactives et de régions protégées de la lumière, ainsi que la mise en réaction d'une région réactive avec un monomère d'acide nucléique activé comportant un groupe de protection photo amovible, ce qui lie ainsi le monomère d'acide nucléique aux régions réactives sur le substrat.

3. Procédé selon la revendication 2, dans lequel le groupe de protection photo amovible attaché au substrat est fixé au substrat par l'intermédiaire d'un agent liant possédant un groupe actif qui est protégé par le groupe de protection photo amovible et qui est disposé au niveau de la terminaison distale dos à la surface du substrat.

4. Procédé selon la revendication 3, dans lequel l'agent liant est sélectionné à partir du groupe constitué d'un nucléoside et d'un nucléotide dont une terminaison 3' est fixée au substrat et dont une terminaison 5' est fixée au groupe de protection photo amovible, ou bien dont une terminaison 5' est fixée au substrat et dont une terminaison 3' est fixée au groupe de protection photo amovible.

5. Procédé selon l'une quelconque des revendications 2 à 4, dans lequel la synthèse et la mise en réaction sont effectuées de manière répétée.

6. Procédé selon l'une quelconque des revendications 1 à 5, dans lequel le premier acide nucléique à simple brin est sélectionné à partir du groupe constitué d'ADN, d'ARN, d'APN et d'une molécule hybride de ceux-ci

7. Procédé selon l'une quelconque des revendications 1 à 6, dans lequel le premier acide nucléique à simple brin présente une longueur d'environ 4 nt à environ 25 nt.

8. Procédé selon l'une quelconque des revendications 1 à 7, comprenant en outre le stockage de la séquence du premier acide nucléique à simple brin et de la position correspondante de la région ponctuelle sur le substrat dans laquelle est immobilisée la séquence.

9. Procédé selon l'une quelconque des revendications 1 à 8, dans lequel le second acide nucléique à simple brin est synthétisé dans un milieu différent du substrat solide dans lequel est synthétisé le premier acide nucléique à simple brin.

10. Procédé selon la revendication 9, dans lequel le second acide nucléique à simple brin comprend de l'ADN complémentaire ou de l'ADN dérivé d'un acide nucléique obtenu par une amplification d'acide nucléique.

11. Procédé selon la revendication 9 ou 10, dans lequel le second acide nucléique à simple brin présente une longueur de 50 nt ou plus, de préférence une longueur d'environ 50 nt à environ 1000 nt.

12. Procédé selon l'une quelconque des revendications 1 à 11, dans lequel l'hybridation comprend en outre l'établissement de la position de la sonde d'acide nucléique à double brin sur le substrat pour qu'elle corresponde à la position du premier acide nucléique à simple brin qui est complémentaire à la première région d'un second acide nucléique à simple brin, second acide nucléique à simple brin qui convertit le premier acide nucléique à simple brin en sonde d'acide nucléique à double brin, et elle comprend le stockage de la position de la sonde d'acide nucléique à double brin.

13. Procédé selon l'une quelconque des revendications 1 à 12, dans lequel l'un du premier acide nucléique à simple brin et de la première région complémentaire du second acide nucléique à simple brin est de l'APN, et l'autre de ceux-ci est de l'ADN ou de l'ARN.
